# EUROPEAN PATENT SPECIFICATION

(11) **EP 0 750 907 B1**
(45) Date of publication and mention of the grant of the patent: **20.03.2002**
(21) Application number: 96304702.2
(22) Date of filing: 26.06.1996
(51) Int. Cl.: A61K 31/365, A61K 39/05, A61K 39/08, A61K 47/02, A61K 47/34

(54) **Stable macrolide and macrolide vaccine compositions**
Stabile Makrolide und Makrolide Imptstoff-Zusammensetzungen
Compositions stables contenant des macrolides et des macrolides combinés avec des vaccins

(30) Priority: 30.06.1995 US 605
(43) Date of publication of application: 02.01.1997
(73) Proprietor: American Cyanamid Company, Madison, New Jersey 07940-0874 (US)
(72) Inventor: Cobb, Ramune Marija, Chipping Norton, New South Wales, 2170 (AU); Schwartzkoff, Christopher Leigh, Turramurra, New South Wales, 2074 (AU)
(74) Representative: Mannion, Sally Kim, Dr.

(56) References cited:
- GB-A- 2 267 707
- UMEHARA O ET AL: "COMPATIBILIDADE ENTRE DORAMECTIN E VACINA CONTRA A FEBRE AFTOSA ADMINISTRADOS SIMULTANEAMENTE EM BOVINOS" REVISTA BRASILEIRA DE PARASITOLOGIA VETERINARIA - BRAZILIAN JOURNAL OF VETERINARY PARASITOLOGY, vol. 2, no. 2, 1993, pages 141-144, XP000653981
- PATENT ABSTRACTS OF JAPAN vol. 012, no. 191 (C-501), 3 June 1988 & JP 62 294623 A (NISSHIN FLOUR MILLING CO LTD), 22 December 1987,

## Description

The present invention relates to vaccine compositions which are stable and to the use of said vaccine compositions for the treatment and control of helminthiasis and infection by acarids and arthropod endo- and ectoparasites in warm-blooded animals, and to a process for the preparation of the vaccine compositions.

Macrolide compounds including macrocyclic lactones such as LL-F28249α-λ compounds, 23-oxo or 23-imino derivatives of LL-F28249α-λ compounds, milbemycin compounds such as milbemycin D, and milbemycin oxime, avermectin compounds such as abamectin, ivermectin and doramectin, and mixtures thereof are useful for the prevention and control of helminthiasis and infection by acarids and arthropod endo- and ectoparasites in warm-blooded animals. Subcutaneous injection of aqueous compositions is one of the preferred methods for administering those compounds.

Vaccines are used to protect warm-blooded animals from a variety of diseases and are also administered by subcutaneous injection. However, a vaccine composition containing both a macrolide compound and antigens is not known. The primary reason for the lack of such a combination is due to the fact that aqueous injectable compositions of macrolide compounds contain dispersing agents which are known to interact with proteins and affect the permeability of the outer membrane of bacterial cells. Such interaction can denature or otherwise disrupt proteins such as antigens.

GB-A-2030043 describes injectable compositions which comprise tetramisole or its levorotatory isomer and a vaccine. However, that application does not disclose a combination vaccine which includes a complex macrolide compound. Further, that application does not describe the use of a dispersing agent, an important component in the aqueous macrolide injectable compositions. Umehara et al. (Rev. Brasil. Parasitol. Vet., 1993, 2(2): 141-144) evaluate possible interferences of the simultaneous administration of a doramectin vaccine and foot-and-mouth disease (FMD) vaccine to cattle. JP-A-62294623 discloses oral compositions comprising antibiotics and deactivated Salmonella. GB-A-2267707 describes macrolides in optional combination with vaccination.

It is therefore an object of the present invention to provide stable vaccine compositions comprising macrolide compounds and antigens. It is also an object to provide stable compositions of macrolide compounds in the absense of an antigen.

It is also an object of the present invention to provide a stable vaccine composition for use in preventing or controlling helminthiasis, infection by acarid and arthropod endo- and ectoparasites and bacterial and viral disease in warm-blooded animals.

It is a further object of the present invention to provide a process for the preparation of stable vaccine compositions.

Surprisingly, it has been found that the vaccine compositions of the present invention are stable in the presence of a dispersing agent and may be stored for prolonged periods of time without loss of antigen and macrolide potency.

Thus, in accordance with the present invention there is provided a vaccine composition for parenteral administration comprising a macrolide compound or mixtures thereof as hereinafter defined, a water soluble organic solvent; a dispersing agent; an adjuvant; at least one antigen; optionally, a preservative, and saline or water or a mixture thereof.

In a preferred aspect of the present invention there is provided a vaccine composition which comprises on a weight to volume basis 0.05% to 2.5% of a macrolide compound or mixtures thereof; 0.1% to 6% of a water soluble organic solvent; 1% to 8% of a dispersing agent; 10% to 50% of an adjuvant; at least one antigen; optionally, up to 0.1% of a preservative; and saline or water or a mixture thereof.

More preferred stable vaccine compositions of the present invention comprise, on a weight to volume basis, 0.1% to 1% of an LL-F28249α-λ compound, a 23-oxo or 23-imino derivative of an LL-F28249α-λ compound, a milbemycin compound, an avermectin compound, or mixtures thereof; 0.2% to 2.5% of a water soluble organic solvent; 2% to 7% of a dispersing agent; 20% to 40% of an adjuvant; at least one antigen; up to 0.1% of a preservative; and saline or water or a mixture thereof.

The macrolide compounds useful in the invention include macrocyclic lactone compounds, milbemycin compounds, avermectin compounds and mixtures thereof described below.

The macrocyclic compounds include but are not limited to those described in U.S. Patent Nos. 5,019,589; 4,886,828; 5,108,992; 5,030,650 and 5,055,486.

The preferred macrocyclic lactone compounds include the compounds designated LL-F28249α-λ which are (collectively) isolates from the fermentation broth of the microorganism *Streptromyces cyaneogriseus* subspecies *noncyanogenus,* deposited in the NRRL (ARS Culture Collection, Fermentation Laboratory, Northern Regional Research Center, U. S. Department of Agriculture, 1815 North University Street, Peoria, IL 61604) under deposit accession No.15773 dated 8 October 1986. The method for preparation of LL-F28249α is disclosed in United States Patent No. 5,106,994 and its continuation, United States Patent No. 5,169,956.

The LL-F28249α-λ compounds are represented by the following structural formula: 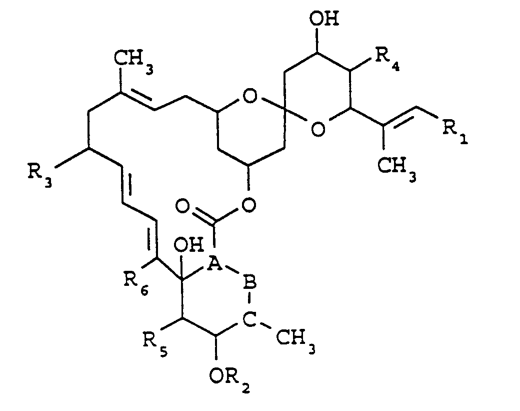 in which R₁, R₂, R₃, R₄, R₅, R₆, A, B and C are as defined in the following table:

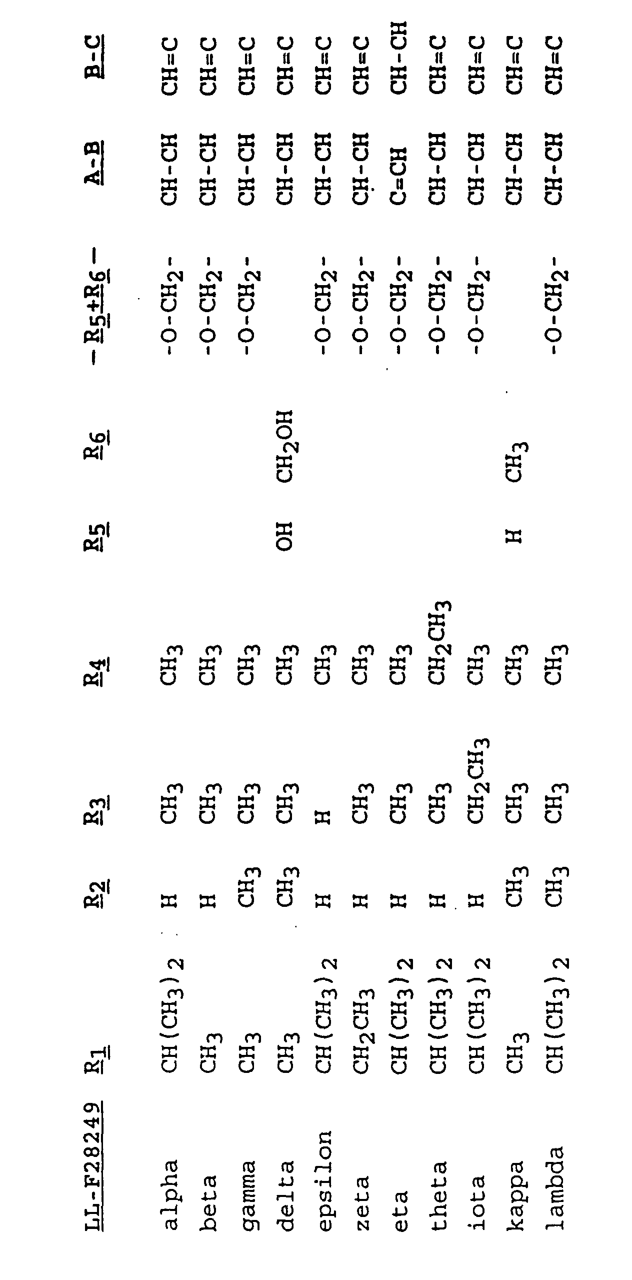

The 23-oxo and 23-imino derivatives of LL-F28249α-λ compounds, useful in the stable vaccine compositions of this invention, are disclosed in United States Patent No. 4,916,154.

A preferred LL-F28249α-λ compound which is useful in the vaccine compositions of this invention is LL-F2824α which has the following structural formula: 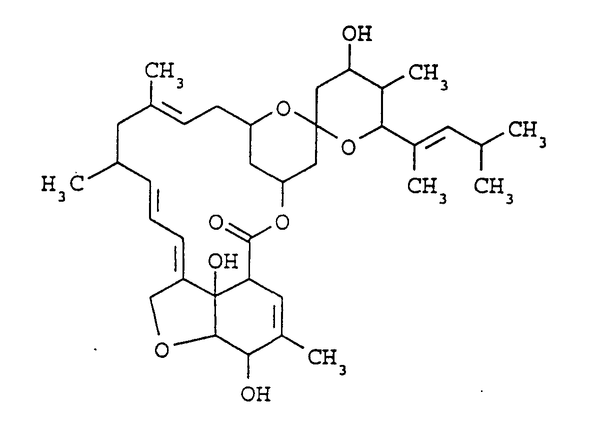 and a preferred derivative of a LL-F28249α-λ compound which is useful in the vaccine compositions of this invention is the 23-imino derivative of LL-F28249α (known as moxidectin) having the following structural formula:

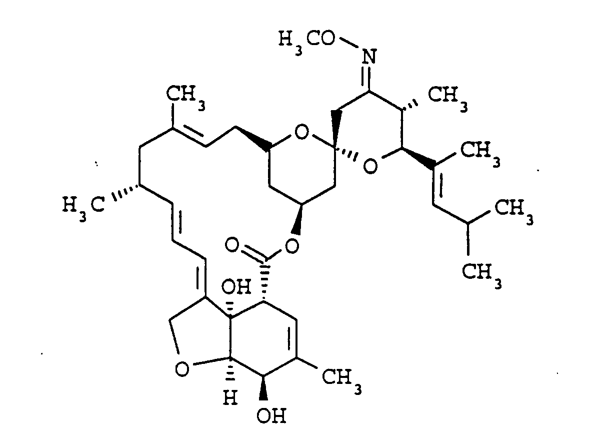

Milbemycin compounds suitable for use in the stable vaccine compositions of this invention include but are not limited to milbemycin D, milbemycin oxime and those compounds described in United States Patent Nos. 3,950,360 and 4,346,171 and 4,547,520. Preferred milbemycin compounds for use in this invention are milbemycin D and milbemycin oxime.

Avermectin compounds which are suitable for use in the invention compositions include but are not limited to abamectin, ivermectin, doramectin and those compounds described in United States Patent Nos. 4,199,569 and 4,310,519, with ivermectin, abamectin and doramectin being preferred. Doramectin and a method for its preparation are described in United States Patent No. 5,089,480.

Antigens suitable for use in the compositions of the present invention include antigens derived from bacterial and viral pathogens of warm-blooded animals including but not limited to those derived by recombinant DNA technology. Preferred antigens include *Clostridium* *perfringens* type *A, B, C* and *D, Clostridium septicum, Clostridium tetani, Clostridium chauvoei, Clostridium novyi* type *B, Clostridium sordelli, Clostridium haemolyticum*, *Pasteurella haemolytica*, *Pasteurella multocida* and *Corynebacterium pseudotuberculosis* which are used in the treatment of diseases such as Lamb dysentery, Pulpy Kidney disease (enterotoxaemia), Malignant Oedema (blood poisoning), Tetanus, Blackleg disease, Black disease, caseous lymphadenitis, and pasteurellosis.

The present invention also provides a process for the preparation of the stable vaccine compositions which comprises:
a) blending a dispersing agent with water to form a first solution;
b) adding to the first solution, a second solution comprising a macrolide compound as defined above or mixtures thereof in a water soluble organic solvent to form a third solution;
c) adding the third solution to a first suspension comprising at least one antigen, an adjuvant and a saline solution to form a second suspension; and
d) adjusting the pH of the second suspension to pH 6 to pH 7.

Dispersing agents useful in the process of the present invention include polyethylene oxide sorbitan mono-oleates such as polyoxyethylene (20) sorbitan mono-oleate (TWEEN®80, Harcros Chemicals), polyoxyethylene alcohols such as laureth 9 and cetomacrogol 1000, sodium lauryl sulfate, dioctyl sodium sulfosuccinate, polyethylene glycols, and α-hydro-ω-hydroxypoly(oxyethylene)poly(oxypropylene)poly(oxyethylene)block copolymers with polyethylene oxide sorbitan mono-oleates such as polyoxyethylene (20) sorbitan mono-oleate being preferred.

Surprisingly, it has now been found that warm-blooded animals which are treated with the vaccine compositions of the present invention (which contain a dispersing agent) respond to vaccination as well as warm-blooded animals treated with traditional vaccine compositions. This is an especially unobvious result because the invention compositions contain a dispersing agent which is generally believed to denature or otherwise disrupt antigens.

Further, it has been found that there is no deleterious effect on the bioavability and performance of the macrolide compound in the compositions of the invention.

The water soluble organic solvent is used to solubilize the macrolide compound. Water soluble organic solvents suitable for use in the present invention include alcohols such as benzyl alcohol, ethanol and methanol, propylene glycols and glycerol formal with benzyl alcohol being preferred.

The adjuvant of the invention is used to stabilize the antigens. Adjuvants suitable for use in the compositions of the present invention include aluminum hydroxide, potassium alum, protamine, aluminum phosphate and calcium phosphate with an aluminum hydroxide gel such as TASGEL® (Pitman-Moore, New Zealand) being preferred.

The pH of the second suspension is preferably adjusted to a pH value of pH 6 to pH 7 with a mineral acid such as sulfuric acid, hydrochloric acid and hydrobromic acid. The pH is adjusted to that range because, in general, at pH values of less than pH 6 or greater than pH 7, denaturing of the antigens may occur.

In a preferred process of the present invention, a preservative is added to the second suspension prior to step (d). Preservatives suitable for use in the present invention include thimerosal ([(o-carboxyphenyl)- thio]ethylmercury sodium salt), formaldehyde, phenol, propylene glycol, glycerol, esters of p-hydroxybenzoic acid, benzoic acid and sodium benzoate with thimerosal being preferred.

In another preferred process of the invention, steps (a) and (b) are conducted at an elevated temperature (>25°C) to ensure that the dispersing agent, macrolide compound and water soluble organic solvent are solubilized.

In the process of this invention, the first solution preferably comprises on a weight basis 10% to 25% of the dispersing agent, the second solution comprises on a weight basis (20% to 40% of the macrolide compound, and the first suspension comprises on a weight basis 25% to 75% of the adjuvant.

The present invention further provides a stable macrolide composition for parenteral administration. The macrolide composition comprises on a weight to volume basis 0.05% to 2.5% of a macrolide compound as defined above or mixtures thereof; 0.1% to 10% of a water soluble organic solvent; 1% to 8% of a dispersing agent; 10% to 50% of an adjuvant and saline or water or a mixture thereof. Preferred macrolide compositions of the present invention comprise on a weight to volume basis, 0.1% to 1% of an LL-F28249α-λ compound, a 23-oxo or 23-imino derivative of an LL-F28249α-λ compound, a milbemycin compound, an avermectin compound or mixtures thereof; 0.2% to 2.5% of a water soluble organic solvent; 2% to 7% of a dispersing agent; 20% to 40% of an adjuvant; at least one antigen; up to 0.1% of a preservative; and saline or water or a mixture thereof.

The stable macrolide compositions of this invention may be prepared by blending a solution of the macrolide compound, water soluble organic solvent, dispersing agent, adjuvant with water or a saline solution.

When parenterally administered, the vaccine compositions of this invention are highly effective for preventing or controlling helminthiasis, infection by acarids and arthropod endo- and ectoparasites and disease in warm-blooded animals such as sheep, cattle, horses, swine, deer, camels, poultry, dogs, cats and goats. Accordingly, the present invention provides a method for preventing or controlling helminthiasis, infection by acarids and arthropod endo- and ectoparasites and disease in warm-blooded animals which comprises parenterally administering to the animals an effective amount of a vaccine composition of the present invention.

Helminthiasis is a widespread disease found in many animals and is responsible for significant economic losses throughout the world. Among the helminths most frequently encountered are the group of worms referred to as nematodes. The nematodes are found in the intestinal tract, heart, lungs, blood vessels and other body tissues of animals and are a primary cause of anemia, weight loss and malnutrition in the infected animals. They do serious damage to the walls and tissue of the organs in which they reside and, if left untreated, may result in death to the infected animals.

The nematodes most commonly found to be the infecting agents of ruminants include Haemonchus and Ostertagia generally found in the abomasum; Cooperia, Trichostrongylus and Nematodirus generally found in the intestinal tract, and Dictyocaulus found in the lungs. In non-ruminant animals important nematodes include Toxocara and Ancylostoma in the intestine and Dirofilaria in the heart of dogs; Ascaris in the intestine of swine; Ascaridia and Heterakis in the intestine of poultry; and large and small strongyles in equines.

Arthropod ectoparasites commonly infecting warm-blooded animals include ticks, mites, lice, fleas, blowfly the ectoparasite *Lucilia sp.* of sheep, biting insects and migrating dipterous larvae such as *Hypoderma sp.* in cattle, *Gastrophilus* in horses and *Cuterebra sp.* in rodents.

Treatment of animals to prevent infestation thereof by the above or to reduce or control the proliferation of these infecting agents in animals is thus an important and desirable advantage of the present invention.

In order to facilitate a further understanding of the invention, the following examples are presented primarily for the purpose of illustrating more specific details thereof.

### EXAMPLE 1

### Preparation of Moxidectin/6 in 1 Vaccine Compositions

The moxidectin/6 in 1 vaccine composition identified as composition number 1 in Table I is prepared by blending TASGEL® (300 mL) with normal saline solution (332.5 mL), adding the appropriate antigen concentrates in the amounts identified below, adding a moxidectin solution (previously prepared by blending a 30%wt/wt moxidectin in benzyl alcohol solution (7.5 mL) with a 17%wt/wt TWEEN®80 solution (300 mL) at about 37°C, and filtering and cooling the resultant solution), adding a 1.3%wt/wt thimerosal solution (7.7 mL), and adjusting the pH to about pH 6.5 with sulfuric acid.

| **Antigen Concentrate** | **Amount (mL)** |
|---|---|
| *Cl. Septicum* | 4.4 |
| *Cl. novyi B* | 1.8 |
| *Cl. tetani* | 1.85 |
| *Cl. perfringens D* | 40.5 |
| *Cl. chauvoei* | 8.75 |
| *C. pseudotuberculosis* | 2.5 |

Using essentially the same procedure, the moxidectin/6 in 1 vaccine compositions identified as composition numbers 2, 3 and 4 in Table I are obtained.

**TABLE I**

| **Moxidectin/6 in 1 Vaccine Compositions** | | | | |
|---|---|---|---|---|
| **Component** | **Amount (%wt/v)** | | | |
| | **Comp. Number 1** | **Comp. Number 2** | **Comp. Number 3** | **Comp. Number 4** |
| Moxidectin (tech.) | 0.23 | 0.44 | 0.25 | 0.53 |
| Benzyl Alcohol | 0.53 | 1.03 | 0.59 | 1.23 |
| TWEEN® 80 | 4.97 | 4.90 | 4.96 | 4.80 |
| Water | 25.04 | 25.33 | 25.14 | 24.39 |
| TASGEL® | 30.00 | 29.53 | 30.00 | 30.00 |
| Normal Saline | 33.25 | 32.87 | 34.52 | 34.50 |
| Thimerosal | 0.01 | 0.01 | 0.01 | 0.01 |
| *Cl. septicum* concentrate | 0.44 | 0.43 | 0.60 | 0.60 |
| *Cl. novyi B* concentrate | 0.18 | 0.18 | 0.67 | 0.67 |
| *Cl. tetani* concentrate | 0.19 | 0.18 | 0.14 | 0.14 |
| *Cl. perfringens D* concentrate | 4.05 | 3.99 | 1.28 | 1.28 |
| *Cl. chauvoei* concentrate | 0.88 | 0.86 | 1.57 | 1.57 |
| *C. pseudotuberculosis* concentrate | 0.25 | 0.25 | 0.26 | 0.26 |

Commercially available 6 in 1 vaccines manufactured by Arthur Webster Pty Limited, Castle Hill, New South Wales, Australia are prepared using essentially the same procedure as described above, except that the moxidectin solution is not used. The 6 in 1 vaccines used in the following examples are identified as composition numbers 5, 6 and 7 in Table II.

**TABLE II**

| **6 in 1 Vaccines** | | | |
|---|---|---|---|
| **Component** | **Amount (%wt/v)** | | |
| | **Composition Number 5** | **Composition Number 6** | **Composition Number 7** |
| Water | 0.76 | 0.71 | 0.94 |
| TASGEL® | 30.00 | 29.97 | 30.00 |
| Normal Saline | 63.25 | 63.33 | 64.50 |
| Thimerosal | 0.01 | 0.01 | 0.01 |
| *Cl. septicum* concentrate | 0.44 | 0.44 | 0.60 |
| *Cl. novyi B* concentrate | 0.18 | 0.18 | 0.67 |
| *Cl. tetani* concentrate | 0.19 | 0.19 | 0.14 |
| *Cl. perfringens D* concentrate | 4.05 | 4.05 | 1.28 |
| *Cl. chauvoei* concentrate | 0.88 | 0.87 | 1.57 |
| *C. pseudotuberculosis* concentrate | 0.25 | 0.25 | 0.26 |

### EXAMPLE 2

### Evaluation of fecal nematode egg counts, seroconversion to clostridial antigens and bodyweights of lambs treated with a moxidectin/6 in 1 vaccine composition

One hundred eighty-five, two to four month old cross-bred merino lambs at weaning which received a primary 6 in 1 vaccination (2 mL of composition number 5 from Table II) six weeks earlier are used in this trial. A single dose (2 mL) of a moxidectin/6 in 1 vaccine (composition number 1 from Table I) is administered to 92. lambs on trial day 0, and 93 lambs receive a single dose (2 mL) of a 6 in 1 vaccine (composition number 5 from Table II). Both treatments are administered subcutaneously high on the right side of the neck.

The lambs are weighed on days 0, 13 and 28 of the trial, at which time fecal samples are taken from the rectum of 15 lambs in both groups. These same lambs are blood sampled on days 0 and 28.

Fecal samples are evaluated for total nematode egg counts by microscopic examination. Pooled serum samples are assayed for antibody against *Clostridium septicum, tetani* and *novyi* type *B* exotoxins by serum neutralization testing in mice. Day 0 (pretreatment) samples from both groups are pooled for antibody analyses. The results are summarized in Tables III, IV and V.

As can be seen from the data in Table III, the moxidectin/6 in 1 vaccine (composition number 1) is highly effective in controlling nematodes. In the group receiving the 6 in 1 vaccine only, the worm burden rises sharply after weaning, while the egg count for the moxidectin/6 in 1 vaccine treated group falls to a low level by the first post-treatment sampling and the count remains low to the final sampling on day 28. This is an especially advantageous finding because the lambs receiving the moxidectin/6 in 1 vaccine maintain a low worm burden despite grazing as one mob on the same contaminated pasture as the 6 in 1 treated group.

Suprisingly, the lambs treated with the moxidectin/6 in 1 vaccine respond as well to vaccination as the lambs treated with the conventional 6 in 1 vaccine (Table IV).

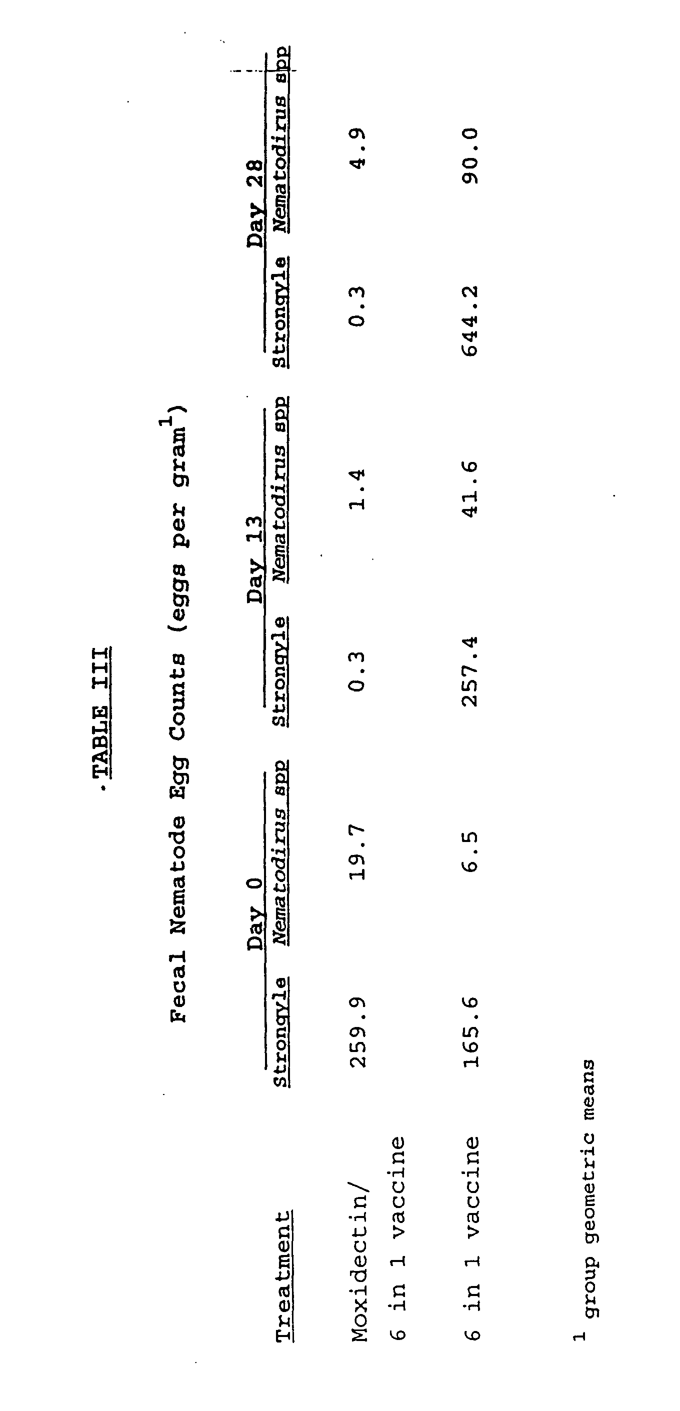

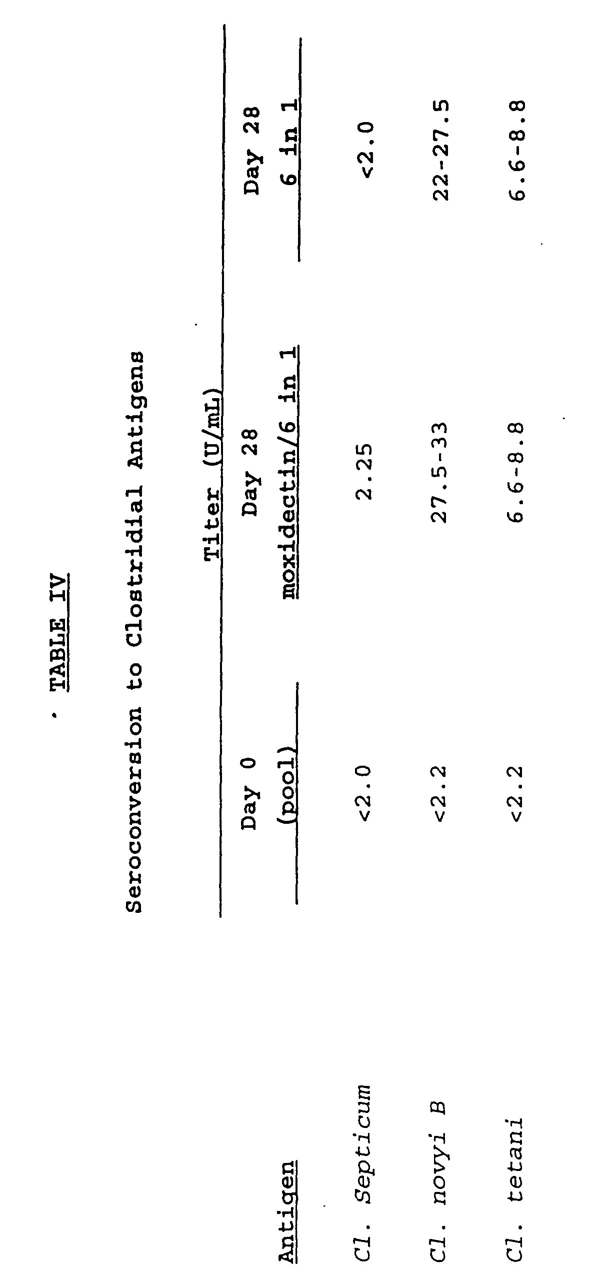

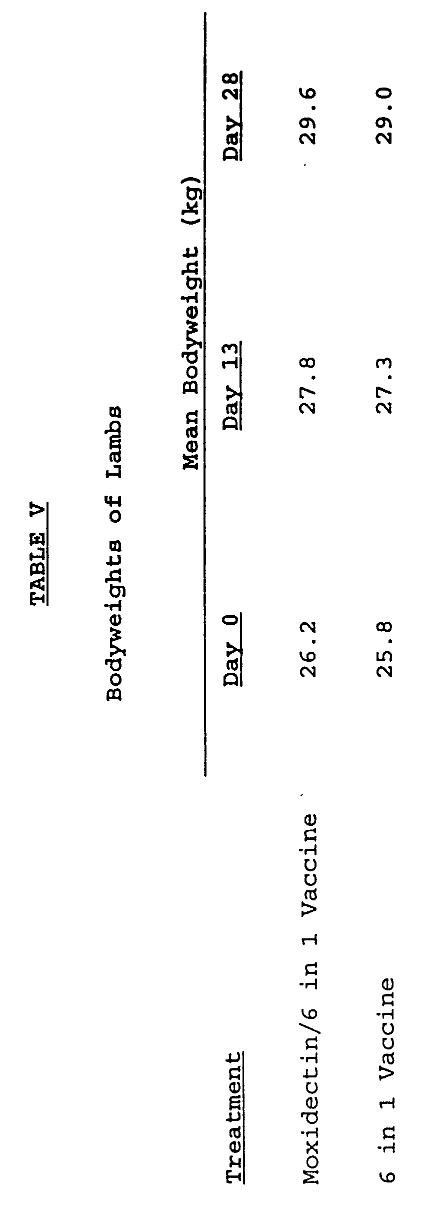

### EXAMPLE 3

### Evaluation of fecal nematode egg counts and seroconversion to clostridial antigens of ewes treated with a moxidectin/6 in 1 vaccine composition.

A group of 188 pregnant merino ewes between 2 and 5 years of age which are due to commence lambing approximately a fortnight after the start of the trial are used. They had been immunized annually with a 6 in 1 vaccine (Glanvac, CSL Ltd.) and had last been drenched two months prior to the trial with levamisole (Nilverm, Coopers Animal Health).

The ewes are weighed at day 0, when their average weight is 50.7 kg, and are randomly allocated into two groups. Ninety-four ewes receive a single dose (2 mL) of a moxidectin/6 in 1 vaccine (composition number 2 from Table I) on trial day 0, while 94 ewes receive a single dose of a 6 in 1 vaccine (composition number 6 from Table II). Fifteen ewes from each group are marked as monitors for fecal and blood sampling on days 0 and 24 of the trial and for a further fecal sample on day 15. Fecal and serum samples are tested as described in Example 2.

Since the trial is undertaken on a commercial property it is not possible to maintain the 6 in 1 treated ewes without nematode treatment beyond trial day 24, so those animals are treated with 7 mL of CYDECTIN® oral drench for sheep (Cyanamid Websters, Castle Hill, New South Wales, Australia) on day 25. No further treatment is administered to the animals that receive the moxidectin/6 in 1 vaccine at day 0. Further fecal samples are taken from the 15 monitor ewes in each group on days 38, 52 and 65 of the trial, and these samples are tested for egg counts as described above.

As can be seen from the data in Table VII, strong antibody responses are shown for both treatments. Advantageously, as can be seen from the data in Table VI, the moxidectin/6 in 1 treatment prevents the classical periparturient nematode egg rise in treated ewes even though they are grazing in pastures with the 6 in 1 treated ewes.

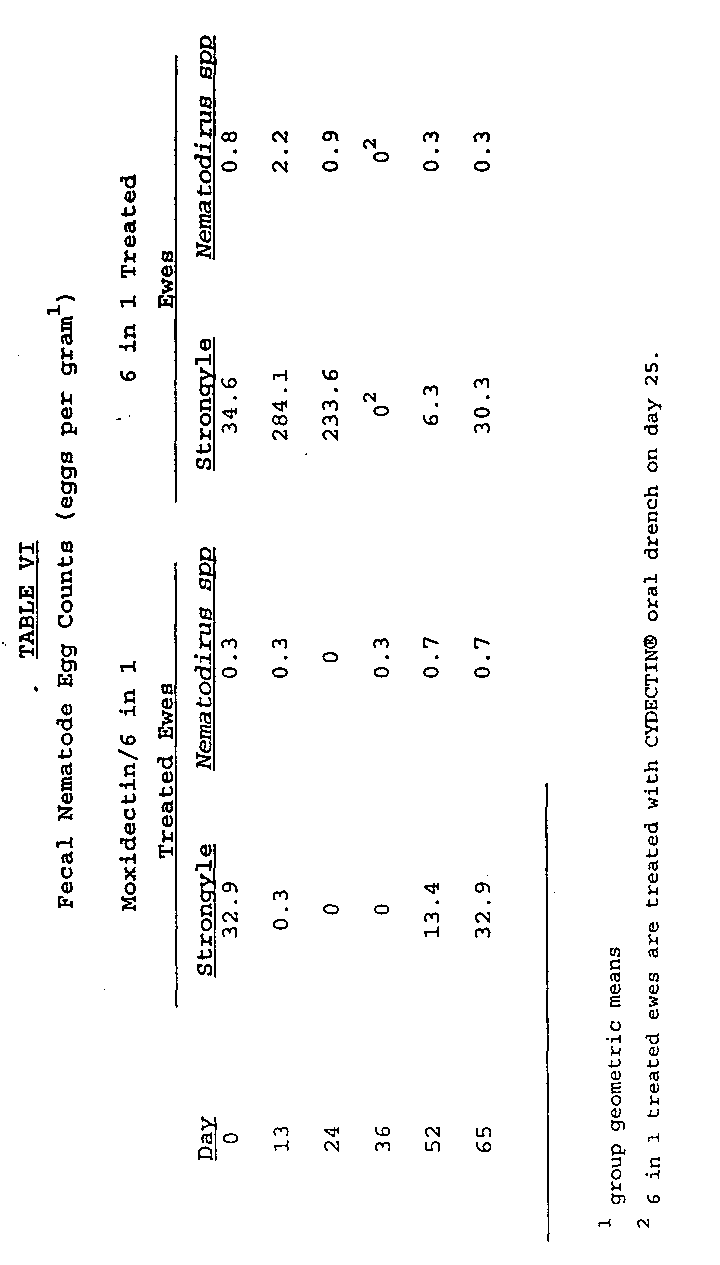

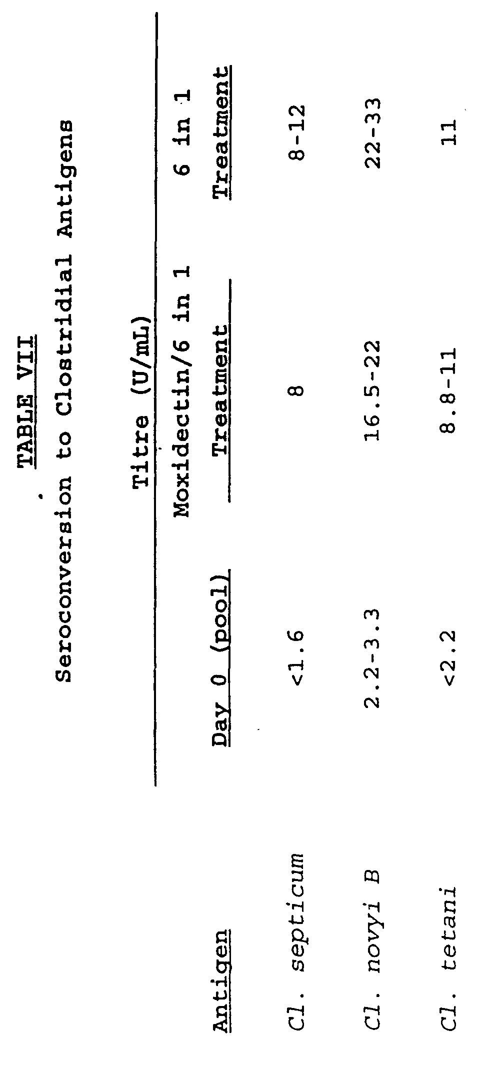

### EXAMPLE 4

### Stability tests of Moxidectin/6 in 1 vaccine compositions

Moxidectin levels and antigen potencies for two of the invention moxidectin/6 in 1 vaccines (composition numbers 3 and 4 from Table I), and antigen potencies for a conventional 6 in 1 vaccine (composition number 7 from Table II) are measured after manufacture, and 6, 12 and 18 months after storage at 4°C. The antigen potencies are measured using the statutory assay procedures described in the British Pharmacopoeia (Veterinary) 1977. Moxidectin levels are determined by HPLC analysis. The results are summarized in Tables VIII, IX and X.

As can be seen from the data in Tables VIII and IX, the antigen potencies and moxidectin levels for composition numbers 3 and 4 remain within the specification requirements. This is an especially surprising discovery because all of the antigen components are proteins, and TWEEN®80 is known to denature proteins.

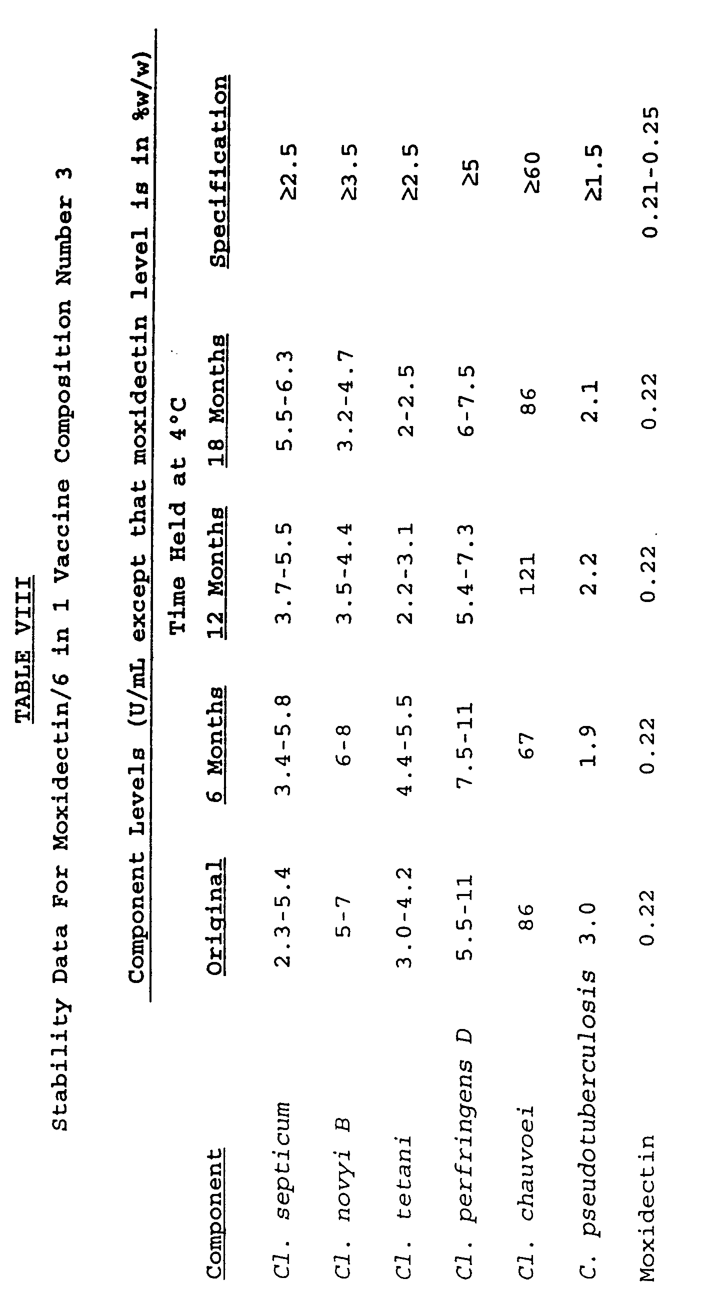

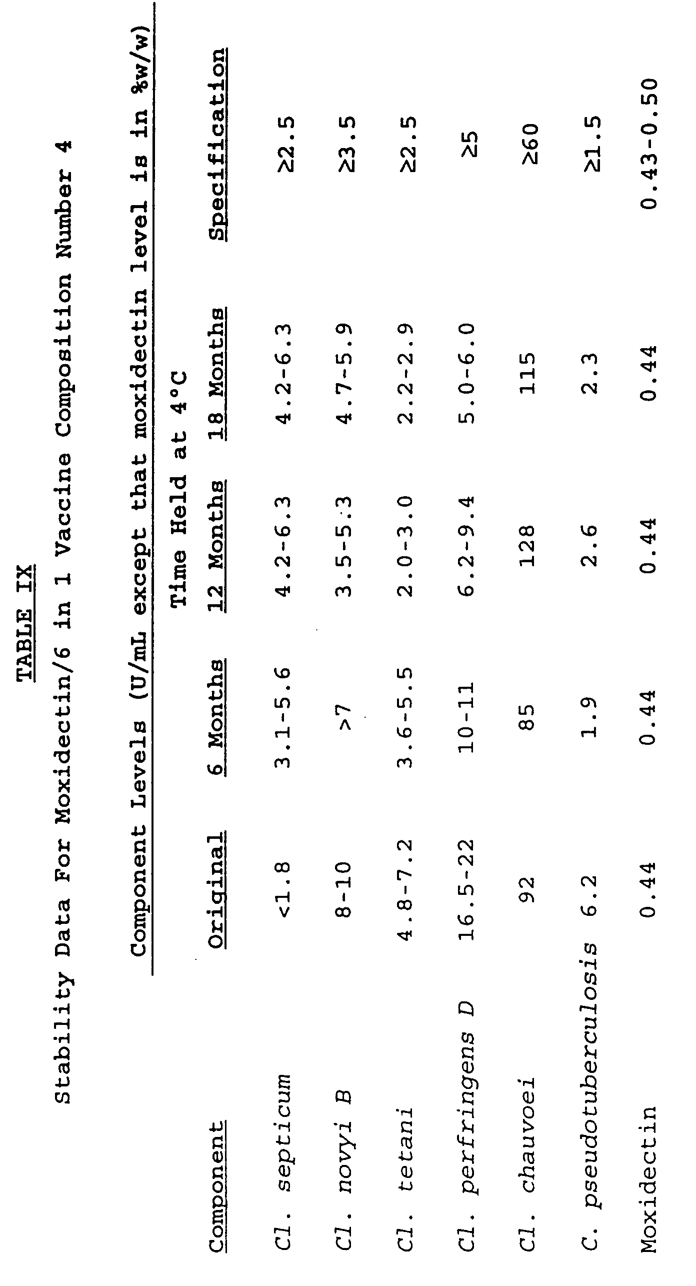

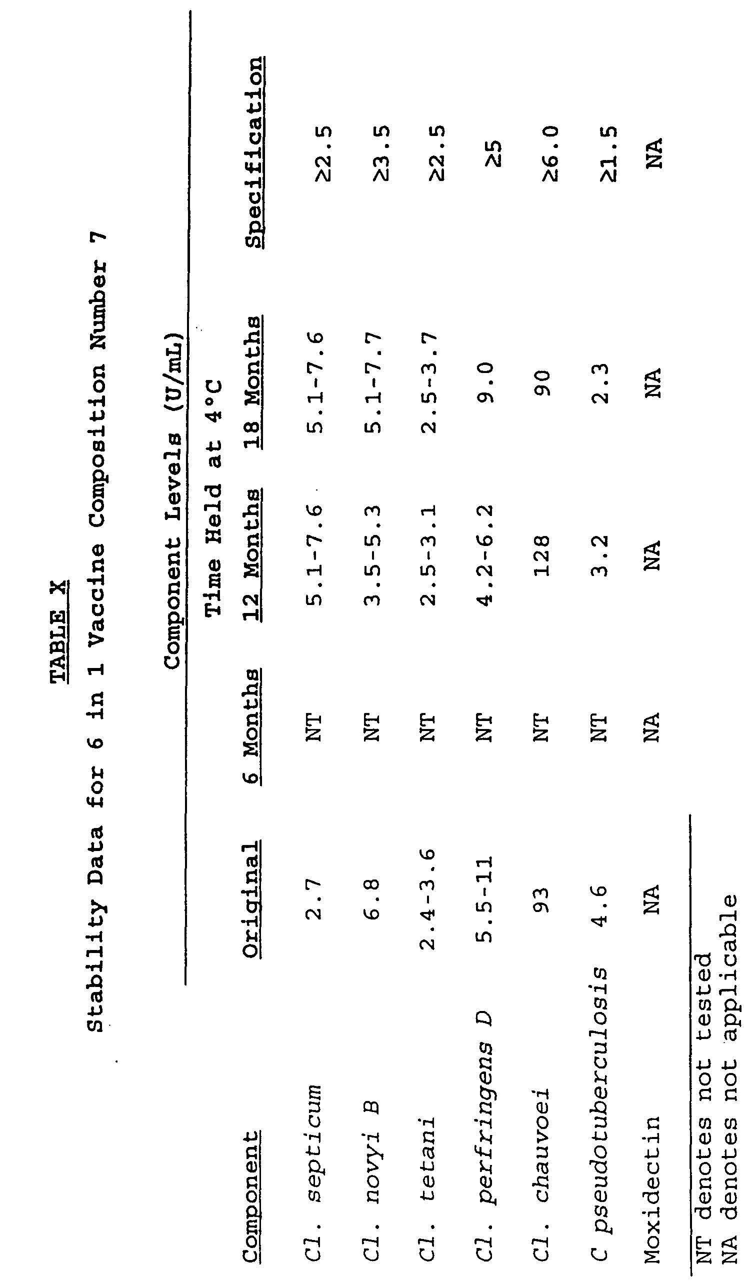

## Claims

1. A vaccine composition for parenteral administration which comprises a macrolide compound or mixtures thereof; a water soluble organic solvent; a dispersing agent; an adjuvant; at least one antigen; and saline or water or a mixture thereof.

2. The vaccine composition according to claim 1 which comprises on a weight to volume basis 0.05% to 2.5% of a macrolide compound or mixtures thereof; 0.1% to 6% of a water soluble organic solvent; 1% to 8% of a dispersing agent; 10% to 50% of an adjuvant; at least one antigen; and saline or water or a mixture thereof.

3. The vaccine composition according to claim 1 which further comprises a preservative.

4. The vaccine composition according to claim 3 which comprises up to 0.1% of a preservative.

5. The vaccine composition according to any of the preceding claims wherein the macrolide compound is selected from the group consisting of an LL-F28249α-λ compound, a 23-oxo or 23-imino derivative of an LL-F28249α-λ compound, a milbemycin, an avermectin and mixtures thereof and the antigen is selected from the group consisting of *Clostridium perfringens* type A, B, C and D, *Clostridium septicum, Clostridium tetani, Clostridium chauvoei, Clostridium novyi* type B, *Clostridium sordelli, Clostridium haemolyticum, Pasteurella haemolytica, Pasteurella multocida* and *Corynebacterium pseudotuberculosis.*

6. The vaccine composition according to claim 5 wherein the macrolide compound is selected from the group consisting of LL-F28249α, moxidectin, milbemycin D, milbemycin oxime, ivermectin, abamectin and doramectin; and the antigen comprises *Clostridium perfringens* type D, *Clostridium septicum, Clostridium tetani, Clostridium chauvoei, Clostridium novyi* type B and *Corynebacterium pseudotuberculosis.*

7. The vaccine composition according to any of the preceding claims which comprises on a weight to volume basis 0.1% to 1% of the macrolide compound or mixtures thereof; 0.2% to 2.5% of the water soluble organic solvent; 2% to 7% of the dispersing agent; and 20% to 40% of the adjuvant.

8. The vaccine composition according any of the preceding claims wherein the water soluble organic solvent is selected from the group consisting of benzyl alcohol, methanol, ethanol, a propylene glycol and glycerol formal; the dispersing agent is selected from the group consisting of a polyethylene oxide sorbitan mono-oleate, a polyoxyethylene alcohol, sodium lauryl sulfate, dioctyl sodium sulfosuccinate, a propylene glycol and an α -hydro-ω-hydroxypoly(oxyethylene)poly(oxypropylene)poly (oxyethylene)block copolymer; the adjuvant is selected from the group consisting of aluminium hydroxide, potassium alum, protamine, aluminium phosphate and calcium phosphate; and the preservative, when present, is selected from the group consisting of thimerosal, formaldehyde, phenol, propylene glycol, glycerol, esters of p-hydroxybenzoic acid, benzoic acid and sodium benzoate.

9. The vaccine composition according to claim 8 wherein the water soluble organic solvent is benzyl alcohol; the dispersing agent is polyoxyethylene (20) sorbitan mono-oleate; the adjuvant is an aluminium hydroxide gel; the preservative is thimerosal; and the macrolide compound is moxidectin.

10. The vaccine composition according any of the preceding claims wherein the pH of the composition is pH 6 to pH 7.

11. A vaccine composition according to any of claims 1 to 10 for use in the treatment of warm blooded animals.

12. Use of a macrolide compound or mixtures thereof or a macrolide composition for the manufacture of a vaccine composition according to any of claims 1 to 10 for preventing or controlling helminthiasis, infection by acarids and arthropod endo-and ectoparasites and disease in warm-blooded animals.

13. A process for the preparation of a vaccine composition according to any of claims 1 to 10 which comprises:
a) blending a dispersing agent with water to form a first solution;
b) adding to the first solution, a second solution which comprises a compound selected from the group consisting of an LL-F28249α-λ compound, a 23-oxo or 23-imino derivative of an LL-F28249α-λ compound, a milbemycin, an avermectin, and mixtures thereof in a water soluble organic solvent to form a third solution;
c) adding the third solution to a first suspension which comprises at least one antigen, an adjuvant and a saline solution to form a second suspension; and
d) adjusting the pH of the second suspension to a pH value of pH 6 to pH 7.

14. The process according to claim 13 wherein the pH is adjusted with a mineral acid.

15. The process according to claim 14 wherein the mineral acid is sulfuric acid.

16. The process according to any of claims 13 to 15 wherein an additional step is added between steps (c) and (d), said additional step comprising adding a preservative to the second suspension.

17. The process according to any of claims 13 to 16 wherein steps (a) and (b) are conducted at an elevated temperature.

18. A macrolide composition for parenteral administration which comprises on a weight to volume basis 0.05% to 2.5% of a macrolide compound or a mixture thereof; 0.1% to 10% of a water soluble organic solvent; 1% to 8% of a dispersing agent; 10% to 50% of an adjuvant and saline or water or a mixture thereof.

19. The macrolide composition according to claim 18 wherein the macrolide compound is selected from the group consisting of an LL-F28249α-λ compound, a 23-oxo or 23-imino derivative of an LL-F28249α-λ compound, a milbemycin compound, an avermectin compound, or mixtures thereof; the water soluble organic solvent is benzyl alcohol; and the adjuvant is aluminium hydroxide gel.

## Revendications

1. Composition de vaccin pour l'administration parentérale qui comprend un composé macrolide ou des mélanges de celui-ci ; un solvant organique soluble dans l'eau ; un agent dispersant ; un adjuvant ; au moins un antigène ; et une solution saline ou de l'eau ou un mélange de celles-ci.

2. Composition de vaccin selon la revendication 1 qui comprend sur une base du poids au volume 0,05% à 2,5% d'un composé macrolide ou de mélanges de celui-ci ; 0,1% à 6% d'un solvant organique soluble dans l'eau ; 1% à 8% d'un agent dispersant ; 10% à 50% d'un adjuvant ; au moins un antigène ; et une solution saline ou de l'eau ou un mélange des celles-ci.

3. Composition de vaccin selon la revendication 1 qui comprend en outre un conservateur.

4. Composition de vaccin selon la revendication 3 qui comprend jusqu'à 0,1% d'un conservateur.

5. Composition de vaccin selon l'une quelconque des revendications précédentes dans laquelle le composé macrolide est choisi parmi le groupe constitué d'un composé de LL-F28249α-λ, d'un dérivé 23-oxo ou 23-imino d'un composé de LL-F28249α-λ, d'une milbémycine, d'une avermectine et des mélanges de ceux-ci et l'antigène est choisi parmi le groupe constitué de *Clostridium perfringens* type A, B, C et D, *Clostridium septicum, Clostridium tetani, Clostridium chauvoei, Clostridium novyi* type B, *Clostridium sordelli, Clostridium haemalyticum, Pasteurella haemolytica, Pasteurella multocida et Corynebacterium pseudotuberculosis.*

6. Composition de vaccin selon la revendication 5 dans laquelle le composé macrolide est choisi parmi le groupe constitué du LL-F28249α, de la moxidectine, de la milbémycine D, de l'oxime de milbémycine, de l'ivermectine, de l'abamectine et de la doramectine ; et l'antigène comprend *Clostridium perfringens* type D, *Clostridium septicum, Clostridium tetani, Clostridium chauvoei, Clostridium novyi* type B *et Corynebacterium pseudotuberculosis*.

7. Composition de vaccin selon l'une quelconque des revendications précédentes qui comprend sur une base de poids au volume 0,1% à 1% du composé macrolide ou des mélanges de celui-ci ; 0,2% à 2,5% du solvant organique soluble dans l'eau ; 2% à 7% de l'agent dispersant ; et 20% à 40% de l'adjuvant.

8. Composition de vaccin selon l'une quelconque des revendications précédentes dans laquelle le solvant organique soluble dans l'eau est choisi parmi le groupe constitué d'alcool benzylique, de méthanol, d'éthanol, d'un propylèneglycol et de glycérolformal ; l'agent dispersant est choisi parmi le groupe constitué d'un monooléate de sorbitan d'oxyde de polyéthylène, un alcool de polyoxyéthylène, du laurylsulfate de sodium, du sulfosuccinate de dioctylsodium, un propylèneglycol et un copolymère bloc de α-hydro-ω-hydroxypoly-(oxyéthylène)poly(oxypropylène)poly(oxyéthylène) ; l'adjuvant est choisi parmi le groupe constitué d'hydroxyde d'aluminium, d'alun de potassium, de protamine, de phosphate d'aluminium et de phosphate de calcium ; et le conservateur, quand il est présent, est choisi parmi le groupe constitué du thimérosal, du formaldéhyde, du phénol, du propylèneglycol, du glycérol, d'esters d'acide p-hydroxybenzoïque, d'acide benzoïque et de benzoate de sodium.

9. Composition de vaccin selon la revendication 8 dans laquelle le solvant organique soluble dans l'eau est l'alcool benzylique ; l'agent dispersant est le monooléate de sorbitan de polyoxyéthylène (20) ; l'adjuvant est un gel d'hydroxyde d'aluminium ; le conservateur est le thimérosal ; et le composé macrolide est la moxidectine.

10. Composition de vaccin selon l'une quelconque des revendications précédentes dans laquelle le pH de la composition est pH 6 à pH 7.

11. Composition de vaccin selon l'une quelconque des revendications 1 à 10 pour l'utilisation dans le traitement des animaux à sang chaud.

12. Utilisation d'un composé macrolide ou des mélanges de celui-ci ou d'une composition de macrolide pour la fabrication d'une composition de vaccin selon l'une quelconque des revendications 1 à 10 pour la prévention ou le contrôle de l'helminthiase, l'infection par les acariens et les endo- et ectoparasites arthropodes et la maladie chez les animaux à sang chaud.

13. Procédé pour la préparation d'une composition de vaccin selon l'une quelconque des revendications 1 à 10 qui comprend :
a) le mélange d'un agent dispersant avec de l'eau pour former une première solution ;
b) l'addition à la première solution d'une seconde solution qui comprend un composé choisi parmi le groupe constitué d'un composé de LL-F28249α-λ, d'un dérivé 23-oxo ou 23-imino d'un composé de LL-F28249α-λ, d'une milbémycine, d'une avermectine et des mélanges de ceux-ci dans un solvant organique soluble dans l'eau pour former une troisième solution ;
c) l'addition de la troisième solution à une première suspension qui comprend au moins un antigène, un adjuvant et une solution saline pour former une seconde suspension ; et
d) l'ajustement du pH de la seconde suspension à une valeur de pH de pH 6 à pH 7.

14. Procédé selon la revendication 13, dans lequel le pH est ajusté avec un acide minéral.

15. Procédé selon la revendication 14, dans lequel l'acide minéral est l'acide sulfurique.

16. Procédé selon l'une quelconque des revendications 13 à 15, dans lequel une étape supplémentaire est ajoutée entre les étapes (c) et (d), ladite étape supplémentaire comprenant l'addition d'un conservateur à la seconde suspension.

17. Procédé selon l'une quelconque des revendications 13 à 16, dans lequel les étapes (a) et (b) sont effectuées à une température élevée.

18. Composition macrolide pour l'administration parentérale qui comprend sur une base du poids au volume 0,05% à 2,5% d'un composé macrolide ou un mélange de celui-ci ; 0,1% à 10% d'un solvant organique soluble dans l'eau ; 1% à 8% d'un agent dispersant ; 10% à 50% d'un adjuvant et une solution saline ou d'eau ou un mélange de celles-ci.

19. Composition macrolide selon la revendication 18, dans laquelle le composé macrolide est choisi parmi le groupe constitué d'un composé de LL-F28249α-λ, d'un dérivé de 23-oxo ou 23-imino d'un composé de LL-F28249α-λ, d'un composé de milbémycine, d'un composé d'avermectine ou des mélanges de ceux-ci ; le solvant organique soluble dans l'eau est l'alcool benzylique ; et l'adjuvant est du gel d'hydroxyde d'aluminium.

## Patentansprüche

1. Impfstoffzusammensetzung zur parenteralen Verabreichung, welche umfasst: eine Makrolidverbindung oder Gemische daraus; ein wasserlösliches organisches Lösungsmittel; ein Dispersionsmittel; einen Hilfsstoff; mindestens ein Antigen und Kochsalzlösung oder Wasser oder ein Gemisch daraus.

2. Impfstoffzusammensetzung gemäß Anspruch 1, welche auf einer Gewicht zu Volumen Basis 0,05% bis 2,5% einer Makrolidverbindung oder Gemische daraus; 0,1% bis 6% eines wasserlöslichen organischen Lösungsmittels; 1% bis 8% eines Dispersionsmittels; 10% bis 50% eines Hilfsstoffs; mindestens ein Antigen und Kochsalzlösung oder Wasser oder ein Gemisch daraus umfasst.

3. Impfstoffzusammensetzung gemäß Anspruch 1, welche ferner ein Konservierungsmittel umfasst.

4. Impfstoffzusammensetzung gemäß Anspruch 3, welche bis zu 0,1% eines Konservierungsmittels umfasst.

5. Impfstoffzusammensetzung gemäß einem der vorhergehende Ansprüche, wobei die Makrolidverbindung ausgewählt wird aus der Gruppe bestehend aus einer LL-F28249α-λ-Verbindung, einem 23-Oxo- oder 23-Imino-Derivat einer LL-F28249α-λ-Verbindung, einem Milbemycin, einem Avermectin und Gemischen daraus, und das Antigen ausgewählt wird aus der Gruppe bestehend aus *Clostridium perfringens* Typ A, B, C und D, *Clostridium septicum, Clostridium tetani, Clostridium chauvoei, Clostridium novyi* Typ B, *Clostridium sordelli, Clostridium haemolyticum, Pasteurella haemolytica, Pasteurella multocida* und *Corynebacterium pseudotuberculosis.*

6. Impfstoffzusammensetzung gemäß Anspruch 5, wobei die Makrolidverbindung ausgewählt wird aus der Gruppe bestehend aus LL-F28249α, Moxidectin, Milbemycin D, Milbemycin oxime, Ivermectin, Abamectin und Doramectin; und das Anitgen *Clostridium perfringens Typ* D, *Clostridium septicum, Clostridium tetani, Clostridium chauvoei, Clostridium novyi* Typ B und *Corynebacterium pseudo-tuberculosis* umfasst.

7. Impfstoffzusammensetzung gemäß einem der vorhergehenden Ansprüche, welche auf einer Gewicht zu Volumen Basis 0,1% bis 1% der Makrolidverbindung oder Gemische daraus; 0,2% bis 2,5% des wasserlöslichen organischen Lösungsmittels; 2% bis 7% des Dispersionsmittels und 20% bis 40% des Hilfsstoffs umfasst.

8. Impfstoffzusammensetzung gemäß einem der vorhergehenden Ansprüche, wobei das wasserlösliche organische Lösungsmittel ausgewählt wird aus der Gruppe bestehend aus Benzylalkohol, Methanol, Ethanol, einem Propylenglycol und Glycerol-Formal; das Dispersionsmittel ausgewählt wird aus der Gruppe bestehend aus einem Polyethylenoxid-sorbitan-mono-oleat, einem Polyoxyethylenalkohol, Natriumlaurylsulfat, Dioctylnatriumsulfosuccinat, einem Propylenglycol und einem α-Hydro-ω-hydroxypoly(oxyethylen)poly(oxypropylen)poly(oxyethylen)-Block-Copoplymer; der Hilfsstoff ausgewählt wird aus der Gruppe bestehend aus Aluminiumhydroxid, Kaliumalaun, Protamin, Aluminiumphosphat und Calciumphosphat; und das Konservierungsmittel, falls vorhanden, ausgewählt wird aus der Gruppe bestehend aus Thimerosal, Formaldehyd, Phenol, Propylenglycol, Glycerol, Estern von p-Hydroxybenzoesäure, Benzoesäure und Natriumbenzoat.

9. Impfstoffzusammensetzung gemäß Anspruch 8, worin das wasserlösliche organische Lösungsmittel Benzylalkohol ist; das Dispersionsmittel Polyoxyethylen(20)sorbitan-mono-oleat ist; der Hilfsstoff ein Aluminiumhydroxid-Gel ist; das Konservierungsmittel Thimerosal ist und die Makrolidverbindung Moxidectin ist.

10. Impfstoffzusammensetzung gemäß einem der vorhergehenden Ansprüche, worin der pH der Zusammensetzung pH 6 oder pH 7 ist.

11. Impfstoffzusammensetzung gemäß einem der Ansprüche 1 bis 10, zur Verwendung bei der Behandlung von warmblütigen Säugern.

12. Verwendung einer Makrolidverbindung oder Gemischen daraus oder einer Makrolidzusammensetzung für die Herstellung einer Impfstoffzusammensetzung gemäß einem der Ansprüche 1 bis 10 zum Verhindern oder Bekämpfen von Helminthiasis, Infektion durch Akariden und Arthropod-Endo- und Ectoparasiten und Erkrankung in warmblütigen Tieren.

13. Verfahren zum Herstellen einer Impfstoffzusammensetzung gemäß einem der Ansprüche 1 bis 10, welches umfasst:
a) Mischen eines Dispersionsmittels mit Wasser, um eine erste Lösung zu bilden;
b) Zugeben zu der ersten Lösung eine zweite Lösung, welche eine Verbindung, ausgewählt aus der Gruppe bestehend aus einer LL-F28249α-λ-Verbindung, einem 23-Oxo- oder 23-Imino-Derivat einer LL-F28249a-λ-Verbindung, einem Milbemycin, einem Avermectin und Gemische daraus in einem wasserlöslichen organischen Lösungsmittel umfasst, um eine dritte Lösung zu bilden;
c) Zugeben der dritten Lösung zu einer ersten Suspension, welche mindestens ein Antigen, einen Hilfsstoff und eine Kochsalzlösung umfasst, um eine zweite Suspension zu bilden; und
d) Anpassen des pH der zweiten Suspension an einen pH-Wert von pH 6 bis pH 7.

14. Verfahren gemäß Anspruch 13, wobei der pH mit einer Mineralsäure angepasst wird.

15. Verfahren gemäß Anspruch 14, wobei die Mineralsäure Schwefelsäure ist.

16. Verfahren gemäß einem der Ansprüche 13 bis 15, wobei ein zusätzlicher Schritt zwischen den Schritten (c) und (d) eingefügt wird, wobei besagter zusätzlicher Schritt das Zugeben eines Konservierungsmitteis zu der zweiten Suspension umfasst.

17. Verfahren gemäß einem der Ansprüche 13 bis 16, wobei Schritte (a) und (b) bei einer erhöhten Temperatur durchgeführt werden.

18. Makrolidzusammensetzung zur parenteralen Verabreichung, welche auf einer Gewicht zu Volumen Basis 0,05% bis 2,5% einer Makrolidverbindung oder ein Gemisch daraus; 0,1% bis 10% eines wasserlöslichen organischen Lösungsmittels; 1% bis 8% eines Dispersionsmittels; 10% bis 50% eines Hilfsstoffs und Kochsalzlösung oder Wasser oder ein Gemisch daraus umfasst.

19. Makrolidzusammensetzung gemäß Anspruch 18, wobei die Makrölidverbindung ausgewählt wird aus der Gruppe bestehend aus einer LL-F28249α-λ-Verbindung, einem 23-Oxo- oder 23-Imino-Derivat einer LL-F28249α-λ-Verbindung, einer Milbemycin-Verbindung, einer Avermectin-Verbindung oder Gemischen daraus; das wasserlösliche organische Lösungsmittel Benzylalkohol ist und der Hilfsstoff Aluminiumhydroxid-Gel ist.
